# EUROPEAN PATENT SPECIFICATION

(11) **EP 2 173 268 B1**
(45) Date of publication and mention of the grant of the patent: **28.09.2011**
(21) Application number: 08728948.4
(22) Date of filing: 04.02.2008
(51) Int. Cl.: A61B 17/88, A61B 17/72

(54) **DEVICES FOR STABILIZING BONE COMPATIBLE FOR USE WITH BONE SCREWS**
VORRICHTUNGEN ZUR STABILISIERUNG VON KOMPATIBLEM KNOCHEN ZUR VERWENDUNG MIT KNOCHENSCHRAUBEN
DISPOSITIFS PERMETTANT DE STABILISER UN OS COMPATIBLE POUR UNE UTILISATION AVEC DES VIS À OS

(30) Priority: 29.06.2007 US 947206 P
(43) Date of publication of application: 14.04.2010
(73) Proprietor: Spinealign Medical, Inc., Pleasanton, CA 94588 (US)
(72) Inventor: CHIRICO, Paul, E., Campbell, CA 95005 (US); CHAN, Benny, M., Fremont, CA 94555 (US); PAKBAZ, Sean, R., San Diego, CA 92109 (US)
(74) Representative: Dee, Ian Mark
(86) International application number: PCT/US2008/052938
(87) International publication number: WO 2009/005851

(56) References cited:
- WO-A-2007/073488
- FR-A- 2 653 006
- US-A1- 2002 026 197
- US-A1- 2004 133 204
- US-A1- 2004 230 193
- US-A1- 2005 070 911
- US-A1- 2007 067 034
- US-A1- 2007 088 436

## Description

### FIELD OF THE INVENTION

Described herein are systems, devices, and methods for treating and supporting bone within a skeletal structure. The invention relates to systems, and devices for treating and supporting cancellous bone within vertebral bodies, including, but not limited to, vertebral bodies affected by osteoporosis.

### BACKGROUND OF THE INVENTION

The use of spinal screws, and particularly pedicle screws, for spinal stabilization has become increasingly popular worldwide. Pedicle screw systems may engage all three columns of the spine and can be used to help resist motion in all planes. Pedicle screws are often used to correct deformity, and/or treat trauma. Similar to other bone screws, pedicle screws may be used in instrumentation procedures to affix rods and plates to the spine. The screws may also be used to immobilize part of the spine to assist fusion by holding bony structures together. Screws may be used to correct deformity, and/or treat trauma.

Several studies suggest that pedicle screw fixation is a safe and effective treatment for many spinal disorders. Standard techniques for pedicle screw placement, however, require extensive tissue dissection to expose entry points and to provide for lateral-to-medial orientation for optimal screw trajectory. Open pedicle fixation and spinal fusion have been associated with extensive blood loss, lengthy hospital stays, and significant cost. Furthermore, it may be particularly difficult to use screws in bone that is disrupted or weakened, as is the case for vertebral compression fractures, particularly those caused by osteoporosis.

US 2004/0230193 discloses a bone fixation device comprising a fixation member having a retracted shape when it is flexible and an expanded shape when it is rigid.

US 2004/0133204 discloses a bone securing device that is adapted to be received in a bone cavity comprising a radially expandable portion.

US 2007/0067034 discloses an expandable stabilisation device that is suitable for deployment in cancellous bone. The device comprises an expandable shaft having a first unexpanded profile and a second expanded profile. The shaft is adapted to cut through cancellous bone during expansion from the first profile to the second profile.

US 2007/0088436 discloses an intra-vertebral bone stent having a tubular member made of a shape memory metal.

US 2002/0026197 discloses an instrument for treating the spine comprising an elongate member having a deformable distal end.

WO 2007/073488 discloses an expandable support device comprising first and second expandable frames.

US 2005/0070911 discloses a device for treating bone comprising a plurality of ribs that can be moved from a collapsed state to an expanded state.

WO 20071067726 discloses an assembly for repositioning of a vertebra comprising an expandable implant, such as a stent, surrounding an expansion device, such as a balloon tipped catheter.

US-5,113,846 discloses an organ manipulator comprising an arrangement of articulated arms that can be moved from a retracted to an expanded configuration.

FR-2,653,006 discloses a centromedullary nail having a tubular body and at least two expansion means.

Described herein are devices, systems and methods for stabilizing bone (particularly spinal bone) that are compatible with the use of bone screws including pedicle screws. In particular, stabilizing implants that are configured to anchor and/or guide bone screws are described herein which may address many of the problems identified above.

### SUMMARY OF THE INVENTION

According to the present invention there is provided a system for stabilising a vertebral body according to claim 1.

Described herein are devices and systems for stabilizing a bone and for supporting one or more bone screws. The devices are self-expanding devices that include an elongate shaft having a plurality of self-expanding struts extendable therefrom, wherein the shaft is adapted to cut through cancellous bone during expansion from a collapsed delivery profile to an expanded deployed profile, and a bone screw attachment region. The self-expanding stabilizing devices (stabilizers) described herein are designed to be implanted into bone, and are configured to cut through bone without substantially compressing the bone. In particular, these devices are configured to cut through cortical bone, without substantially compressing it. They are also configured to be left within the bone (e.g., implanted).

The shaft is typically adapted to be positioned within cancellous bone and has an expanded deployed profile and a collapsed delivery profile. The bone screw attachment region may be a threaded region that is configured to mate to the threading of a bone screw. In some variations, the bone screw attachment region includes a collapsible or crushable region into which the bone screw, anchor, and/or connector may be inserted. In some variations the bone screw is a multi-part bone screw, having multiple parts that can be attached to this attachment region collectively or separately (or both). In some variations, the bone screw attachment region is a post onto which the bone screw (or a portion or component of a bone screw) slides or screws. For example a bone screw attachment region may be a post onto which the bone screw coaxially connects.

In some variations, the bone screw attachment region includes a threaded region located at one end of the elongate shaft. The stabilization devices may also have more than one attachment region for a bone screw. For example, a stabilization device may include a bone screw attachment region at the proximal and also at the distal end. The same bone screw may be connected or attached to both attachment regions. In some variations, different bone screws may be attached to each attachment region.

Any appropriate self-expandable stabilization device may include a bone screw attachment region. For example, in some variations, the stabilization devices may be configured to self-expand in bone and to support one or more bone screws. For example, a stabilization device may include an elongate shaft having two or more continuous curvature of bending struts, wherein the struts extend from the shaft more in the deployed configuration than in the delivery configuration, a proximal region having a first releasable attachment configured to attach to an inserter, a distal region having a second releasable attachment configured to attach to the inserter, and a bone screw attachment region configured to secure to a bone screw.

As mentioned above, the bone screw attachment region may be a threaded region (e.g., configured so that a threaded bone screw such as a pedicle screw may mate with the bone screw attachment region). For example, the bone screw attachment region may be a threaded opening at the proximal end of the stabilization device. In some variations the releasable attachment configured to attach an inserter is also configured as the bone screw attachment region.

The stabilization device may include struts that are formed of a shape memory alloy (e.g., a nickel titanium alloy). In some variations, the first releasable attachment comprises an L-shaped notch. The stabilization device may also include one or more releasable attachment regions for use in inserting or releasing the devices. For example, the devices may include a first releasable attachment that comprises a threaded region. This releasable attachment may also be configured as a bone screw attachment region. In some variations, the second releasable attachment comprises an L-shaped notch.

The maximum distance between the struts of the self-expanding device at a point along the length of the shaft in the expanded deployed configuration maybe between about 0.5 and about 30 mm. In some variations, the maximum distance between the struts at a point along the length of the shaft in the expanded deployed configuration is between about 8 and about 20 mm. The maximum distance between the struts at a point along the length of the shaft in the expanded deployed configuration may be about 10 mm. The maximum distance between the struts at a point along the length of the shaft in the expanded deployed configuration maybe about 18 mm. The shaft may also be adapted to abut a surface of cortical bone adjacent the cancellous bone without passing there through

In some variations the system includes a bone screw. Any appropriate bone screw may be used. For example, the bone screw may be a pedicle screw. The bone screw may be any size, including commercially available sizes, and may be threaded to any thread size (e.g., having any minimum, maximum, and/or pitch).

One variation of a bone screw that maybe used is a two-part bone screw that includes a first bone screw assembly having an elongate shaft that is threaded along the outer surface of the shaft, and a second bone screw assembly configured to mate over the first bone screw assembly, wherein the second bone screw assembly has a threaded outer surface configured to contact bone, and a threaded inner channel that is configured to thread onto the outer surface of the first bone screw assembly. The bone screws described herein (which are compatible with the stabilization devices described herein, but may be used independently of them), including the multi-part bone screws, may be attached or secured by the stabilization device after it has been inserted into the bone and detached from the inserter (applicator). In some variations, the bone screw (or one component of a bone screw) is pre-attached to the implant before it is positioned in the bone. For example, once the stabilization device is detached from the inserter or applicator a bone screw or one component of a bone screw can be attached to the stabilization device. In one variation, when the inner component (e.g., the inner pin) of the multi-part bone screw is attached to the stabilization device, the second (e.g., outer) component can then be attached over the inner component by screwing in place. Alternatively, the outer screw component can fit snugly over the inner component when the inner component has a smooth surface (having approximately the same diameter as the delivery device for the bone stabilizing device, for example).

The pitch of the threads on the outer surface of the first bone screw assembly may be greater than the pitch of the threads on the outer surface of the second bone screw assembly. In some variations the pitch of the threads on the outer surface of the first bone screw assembly is less than (or equal to) the pitch of the threads on the outer surface of the second bone screw assembly. In some variations, the size of the threads on the outer surface of the second bone screw assembly (or outer bone screw assembly) is greater than the size of the threads on the outer surface of the first bone screw assembly (or inner bone screw assembly). In some variations, only one (e.g., only the outer or only the inner) component of a multi-part bone screw assembly is threaded. For example, the inner bone screw assembly may be smooth on the surface or face of the screw engaging the outer bone screw assembly.

The second bone screw assembly may be a tube that is threaded on both the inside and the outside. This tube may be completely hollow (e.g., pass through the length of the second bone screw assembly), and may be threaded along the entire length of its inner surface. The distal portion of the outer bone screw assembly may include a head region.

The inner bone screw may come in a variety of sizes, and the portion that remains in the body may be attached to a longer (e.g., delivery) shaft. This long delivery shaft can be removed and attached from the inner screw (e.g., by unscrewing). This could allow the inner screw to be inserted into the stabilization device by following the same pathway as the stabilization device. The screw can therefore be maneuvered and at least partially secured in position or attached to the stabilization device.

In some variations, a three-part screw (e.g., pedicle screw) may be used as a multi-part screw or system. For example, the screw may be an assembly including an outer screw component, an inner screw component, and a third screw component that may also be threaded. This third component may be an adaptable head that can secure the other two components together, or it may be used to link to other structures, including rods (connecting to other screws), screws or the like.

In some variations the inner screw is partially threaded (e.g., the part that attaches to the spine and stabilizing device), and the outer screw can be placed over this inner screw and snugly fitted on until a third screw attaches the two (first and second components) together. The inner screw can also have threads all the way to the back and also be attached to the outer screw by a third smaller screw or adaptable head.

In some variations, the multi-part screws (or attachment systems) are configured to be used with a cannula and/or a guidewire. For example, one or more components of the multi-part screw can be threaded over a guidewire (which may also be referred to as a pin wire in this context). The stabilization implants described herein may also be used with a cannula by configuring them appropriately. For example, they may have a diameter appropriate for use with a cannula, and may include a guidewire passage or rapid exchange port for use with a guidewire.

In some variations the inner screw has a conical shape so that it could be readily positioned back into the stabilizing device if it is exchanged.

The stabilizing device can be anchored into the cancellous bone using methacrylate prior to insertion of the pedicle screw (e.g., an inner or outer screw of a multi-part screw), or after insertion of the screw. Alternatively, when multi-part screw is used, the first screw component can be placed, and then methacrylate can be added. Thus, a cement can be used at any appropriate time during insertion.

Any of the systems for stabilizing a bone and for placing a bone screw may also include an introducer for inserting the stabilization device, a handle for inserting the device, and a trocar and/or a twist drill for penetrating the tissue to position the device. The system may also include a bone cement. In some variations, the systems also includes a cement cannula for delivering the cement.

Also described herein are methods not claimed, of treating a bone that include delivering the self-expanding device within a cancellous bone (wherein the device has an elongate shaft, a bone screw attachment region, and a plurality of struts extending therefrom), and securing a bone screw in the bone screw attachment region of the device. In some variations, the method also includes the step of allowing the device to expand within the cancellous bone so that a cutting surface of the device cuts through the cancellous bone.

The method may also include the step of visualizing the device within the bone, and/or drilling a hole into the cancellous bone through which the self-expanding device may be inserted. Force may be applied to further expand the device within the cancellous bone. A bone cement may be applied within the cancellous bone.

### BRIEF DESCRIPTION OF THE DRAWINGS

FIG. 1 is a lateral view of a normal human spinal column. FIG. 1B is a superior view of a normal human lumbar vertebra. FIG. 1C is a lateral view of a functional spinal unit having two vertebral bodies and an intervertebral disc. FIG. 1D is a posterolateral oblique view of a vertebra. FIG. 1E illustrates a portion of a spine wherein a vertebral body is fractured; FIG. 1F illustrates a human body with the planes of the body identified.

FIGS. 2A-2C are variations of pedicle screws.

FIGS. 2D and 2E show a two-part bone screw, which may be configured as a pedicle screw.

FIGS. 3A and 3B are enlarged side and side perspective views (respectively) of the stabilization device shown in FIG. 2A.

FIGS. 4A and 4B are enlarged side and side perspective views (respectively) of the stabilization device shown in FIG. 2C.

FIG. 5A is a stabilization device to which a pedicle screw is attached.

FIG. 5B shows two implanted stabilization device and pedicle screws.

FIG. 5C is a stabilization device to which another variation of a pedicle screw is attached.

FIG. 6A is one variation of a stabilization device removably attached to an inserter.

FIG. 6B is another variation of a stabilization device removably attached to an inserter.

FIGS. 7A-7J illustrate one method of treating a bone.

FIG. 8 is a schematic flowchart illustrating one method of treating a bone using the stabilization devices described herein.

### DETAILED DESCRIPTION OF THE INVENTION

The devices and systems described herein may aid in the treatment of fractures and microarchitetcture deterioration of bone tissue, including vertebral compression fractures ("VCFs") or other indications including those arising from osteoporosis. The implantable stabilization devices described herein (which may be referred to as "stabilization devices" or simply "devices") may help restore and/or augment bone. Thus, the stabilization devices described herein may be used to treat pathologies or injuries. For purposes of illustration, many of the devices, systems and methods described herein are shown with reference to the spine. However, these devices, systems and methods may be used in any appropriate body region, particularly bony regions. For example, the methods, devices and systems described herein may be used to treat hip bones.

Although examples of the use of the devices described herein illustrate the use of these devices and methods to treat compression fractures, it should be understood that these methods and devices are not limited to treatment of compression fractures or to treatment of damage arising from osteoporosis. For example, the methods and device described herein may be used to treat bone damage arising from other disease states (e.g., cancer, tumors, infection, etc.) and non-disease states.

The devices described herein are self-expanding bone stabilization devices that may support one or more bone screws. Thus, a bone stabilization device may be inserted into a bone (e.g., a vertebral bone) and a bone screw may then be inserted into the stabilization device. In some variations the bone screw is inserted with the self-expanding stabilization device.

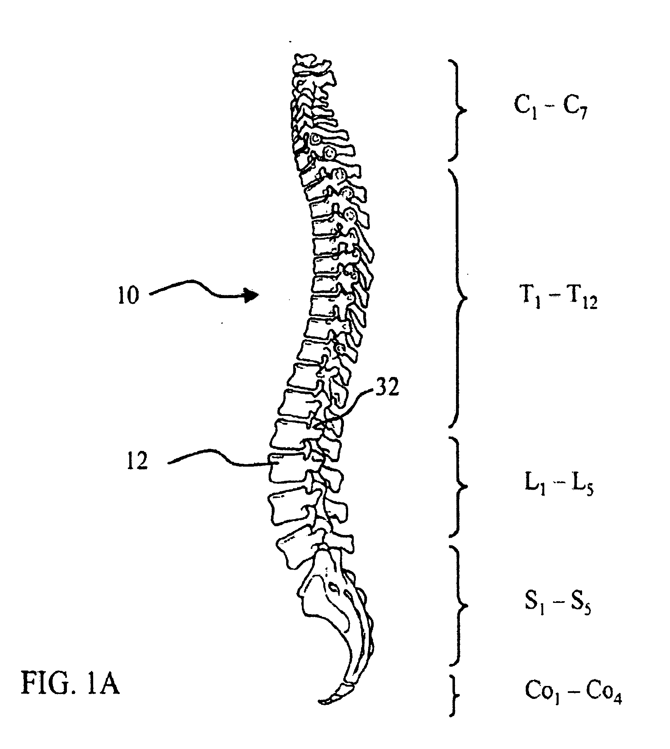

By way of background the anatomy of the spine will briefly be described with reference to FIGS. 1A-1F, followed by a description of the bone stabilization devices (and variations of such devices) that may be used in any of these anatomical regions.

The human spinal column 10, as shown in FIG. 1A, is comprised of a series of thirty-three stacked vertebrae 12 divided into five regions. The cervical region includes seven vertebrae, known as C1-C7. The thoracic region includes twelve vertebrae, known as T1-T12. The lumbar region contains five vertebrae, known as Ll-L5. The sacral region is comprised of five fused vertebrae, known as S1-S5, while the coccygeal region contains four fused vertebrae, known as Co1-Co4.

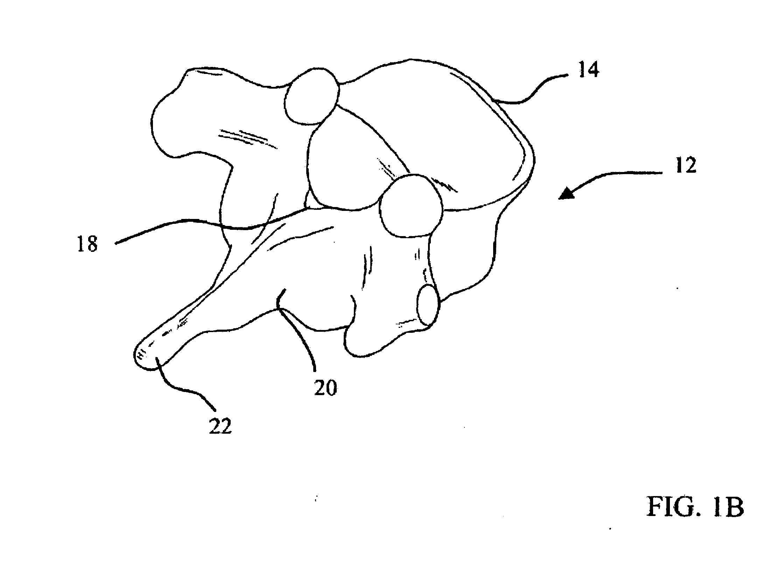

An example of one vertebra is illustrated in FIG. 1B, which depicts a superior plan view of a normal human lumbar vertebra 12. Although human lumbar vertebrae vary somewhat according to location, the vertebrae share many common features. Each vertebra 12 includes a vertebral body 14. Two short boney protrusions, the pedicles, extend dorsally from each side of the vertebral body 14 to form a vertebral arch 18 which defines the vertebral foramen.

At the posterior end of each pedicle, the vertebral arch 18 flares out into broad plates of bone known as the laminae 20. The laminae 20 fuse with each other to form a spinous process 22. The spinous process 22 provides for muscle and ligamentous attachment. A smooth transition from the pedicles to the laminae 20 is interrupted by the formation of a series of processes. Two transverse processes thrust out laterally, one on each side, from the junction of the pedicle with the lamina 20. The transverse processes serve as levers for the attachment of muscles to the vertebrae 12. Four articular processes, two superior and two inferior, also rise from the junctions of the pedicles and the laminae 20. The superior articular processes are sharp oval plates of bone rising upward on each side of the vertebrae, while the inferior processes 28, 28' are oval plates of bone that jut downward on each side.

The superior and inferior articular processes each have a natural bony structure known as a facet. The superior articular facet faces medially upward, while the inferior articular facet faces laterally downward. When adjacent vertebrae 12 are aligned, the facets, capped with a smooth articular cartilage and encapsulated by ligaments, interlock to form a facet joint 32. The facet joints are apophyseal joints that have a loose capsule and a synovial lining.

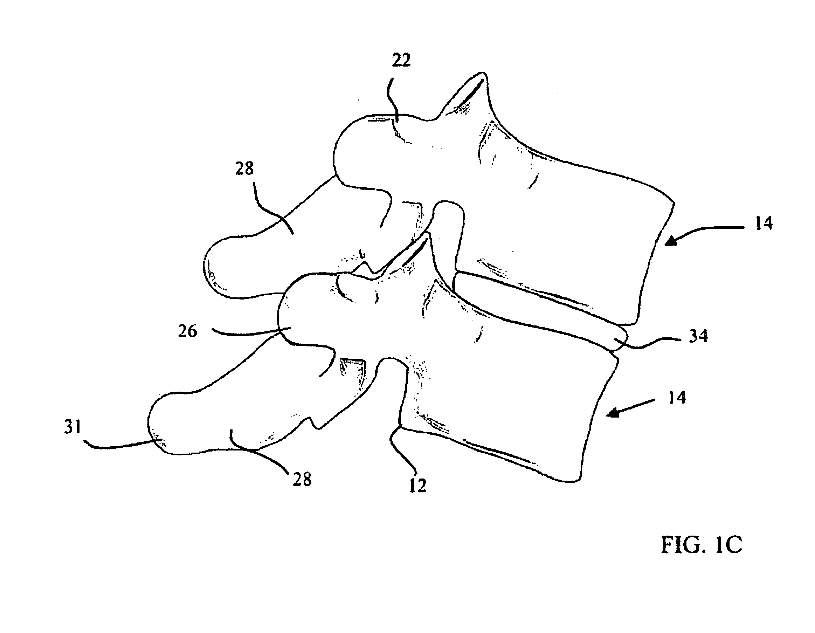

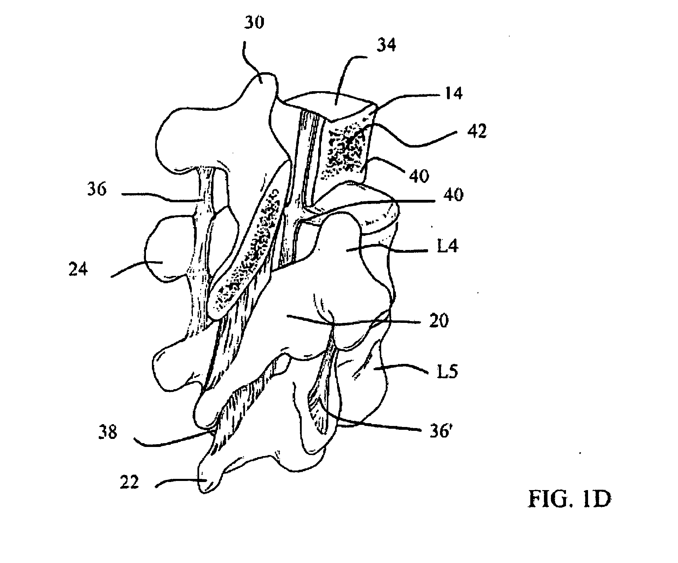

An intervertebral disc 34 between each adjacent vertebra 12 (with stacked vertebral bodies shown as 14, 15 in FIG. 1C) permits gliding movement between the vertebrae 12. The structure and alignment of the vertebrae 12 thus permit a range of movement of the vertebrae 12 relative to each other. FIG. 1D illustrates a posterolateral oblique view of a vertebra 12. The vertebral body 14 is shown in a cut-away that illustrates the cortical bone 40 which forms the exterior of the bone (in this case the vertebral body) and the spongy cancellous bone 42 located within the interior of the cortical bone.

Despite the small differences in mineralization, the chemical composition and true density of cancellous bone are similar to those of cortical bone. As a result, the classification of bone tissue as either cortical or cancellous is based on bone porosity, which is the proportion of the volume of bone occupied by non-mineralized tissue. Cortical bone has a porosity of approximately 5-30% whereas cancellous bone porosity may range from approximately 30 to more than 90%. Although typically cortical bone has a higher density than cancellous bone, that is not necessarily true in all cases. As a result, for example, the distinction between very porous cortical bone and very dense cancellous bone can be somewhat arbitrary.

The mechanical strength of cancellous bone is well known to depend on its apparent density and the mechanical properties have been described as those similar to man-made foams. Cancellous bone is ordinarily considered as a two-phase composite of bone marrow and hard tissue. The hard tissue is often described as being made of trabecular "plates and rods." Cancellous microstructure can be considered as a foam or cellular solid since the solid fraction of cancellous bone is often less than 20% of its total volume and the remainder of the tissue (marrow) is ordinarily not significantly load carrying. The experimental mechanical properties of trabecular tissue samples are similar to those of many man-made foams. If a sample of tissue is crushed under a prescribed displacement protocol, the load-displacement curve will initially be linear, followed by an abrupt nonlinear "collapse" where the load carrying capacity of the tissue is reduced by damage. Next follows a period of consolidation of the tissue where the load stays essentially constant, terminated by a rapid increase in the load as the tissue is compressed to the point where the void space is eliminated. Each of the mechanical properties of cancellous bone varies from site-to-site in the body. The apparent properties of cancellous bone as a structure depend upon the conformation of the holes and the mechanical properties of the underlying hard tissue composing the trabeculae. The experimental observation is that the mechanical properties of bone specimens are power functions of the solid volume fraction. The microstructural measures used to characterize cancellous bone are very highly correlated to the solid volume fraction. This suggests that the microstructure of the tissue is a single parameter function of solid volume fraction. If this is true, the hard tissue mechanical properties will play a large role in determining the apparent properties of the tissue. At this time, little is known about the dependence of trabecular hard tissue mechanical properties on biochemical composition or ultrastructural organization.

Cancellous bone in the joints and spine is continuously subject to significant loading. One consequence of this is that the tissue can experience, and occasionally accumulate, microscopic fractures and cracks. These small damages are similar to those seen in man-made materials and are, in many cases, the result of shear failure of the material. It is known that microcracks accumulate with age in the femoral head and neck, leading to a hypothesis that these damages are related to the increase in hip fracture with age. However, no such association of increased crack density with age was found in human vertebral cancellous bone despite the high incidence of spinal fractures, particularly in women.

Adult cortical and cancellous bone can be considered as a single material whose apparent density varies over a wide range. The compressive strength of bone tissue is proportional to the square of the apparent density. Cortical bone morphology and composition can be characterized by an examination of microstructure, porosity, mineralization, and bone matrix. These parameters seldom vary independently but are usually observed to vary simultaneously. Mechanical properties vary through the cortical thickness due to variations in microstructure, porosity, and chemical composition.

Mechanical properties are dependent on microstructure. The strongest bone type is circumferential lamellar bone, followed in descending order of strength by primary laminar, secondary Haversian, and woven-fibered bone. All normal adult cortical bone is lamellar bone. Most of the cortical thickness is composed of secondary Haversian bone. Circumferential lamellar bone is usually present at the endosteal and periosteal surfaces. In the adult, woven-fibered bone is formed only during rapid bone accretion, which accompanies conditions such as fracture callus formation, hyperparathyroidism, and Paget's disease.

Aging is associated with changes in bone microstructure which arc caused primarily by internal remodeling throughout life. In the elderly, the bone tissue near the periosteal surface is stronger and stiffer than that near the endosteal surface due primarily to the porosity distribution through the cortical thickness caused by bone resorption. Bone collagen intermolecular cross-linking and mineralization increase markedly from birth to 17 years of age and continue to increase, gradually, throughout life. Adult cortical bone is stronger and stiffer and exhibits less deformation to failure than bone from children. Cortical bone strength and stiffness are greatest between 20 and 39 years of age. Further aging is associated with a decrease in strength, stiffness, deformation to failure, and energy absorption capacity.

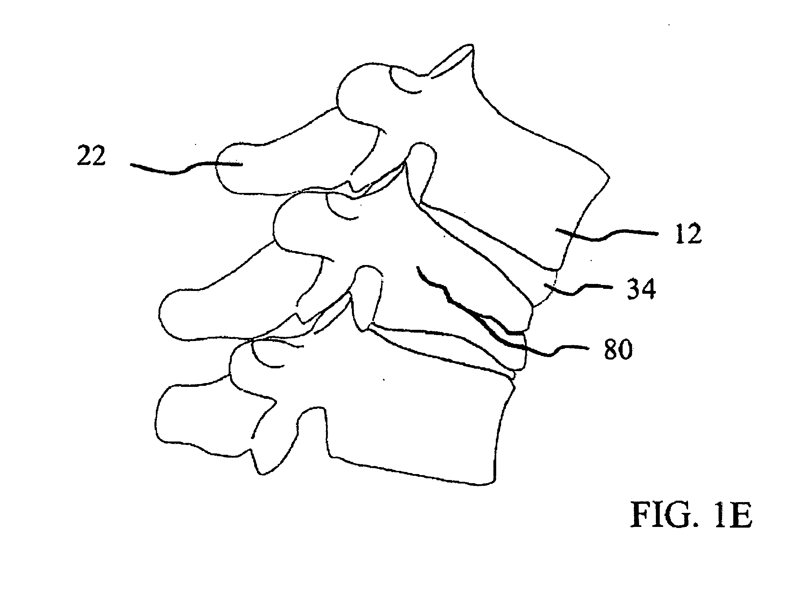

From this understanding of bone, it can be appreciated that when a vertebral body becomes damaged, as illustrated in FIG. 1E, such as when a fracture 80 occurs, a portion of the vertebral body typically collapses. This collapse can occur as a result of micro-architecture deterioration of the bone tissue.

The terms caudal and cephalad may be used in conjunction with the devices and operation of the devices and tools herein to assist in understanding the operation and/or position of the device and/or tools.

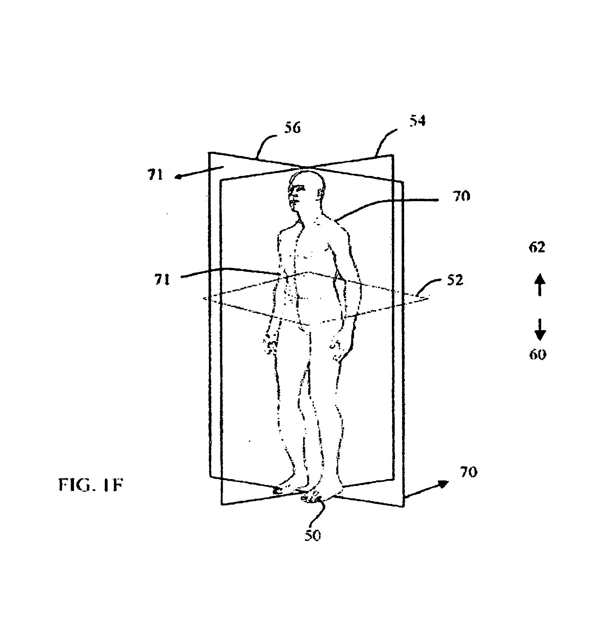

In order to understand the configurability, adaptability, and operational aspects of the devices disclosed herein, it is helpful to understand the anatomical references of the body 50 with respect to which the position and operation of the devices, and components thereof, are described. There are three anatomical planes generally used in anatomy to describe the human body and structure within the human body: the axial plane 52, the sagittal plane 54 and the coronal plane 56 (see FIG. 1F). Additionally, devices and the operation of devices and tools are better understood with respect to the caudad 60 direction and/or the cephalad direction 62. Devices and tools can be positioned dorsally 70 (or posteriorly) such that the placement or operation of the device is toward the back or rear of the body. Alternatively, devices can be positioned ventrally 72 (or anteriorly) such that the placement or operation of the device is toward the front of the body. Various embodiments of the devices, systems and tools of the present invention may be configurable and variable with respect to a single anatomical plane or with respect to two or more anatomical planes. For example, a component may be described as lying within and having adaptability or operability in relation to a single plane. For example, a device may be positioned in a desired location relative to an axial plane and may be moveable between a number of adaptable positions or within a range of positions. Similarly, the various components can incorporate differing sizes and/or shapes in order to accommodate differing patient sizes and/or anticipated loads.

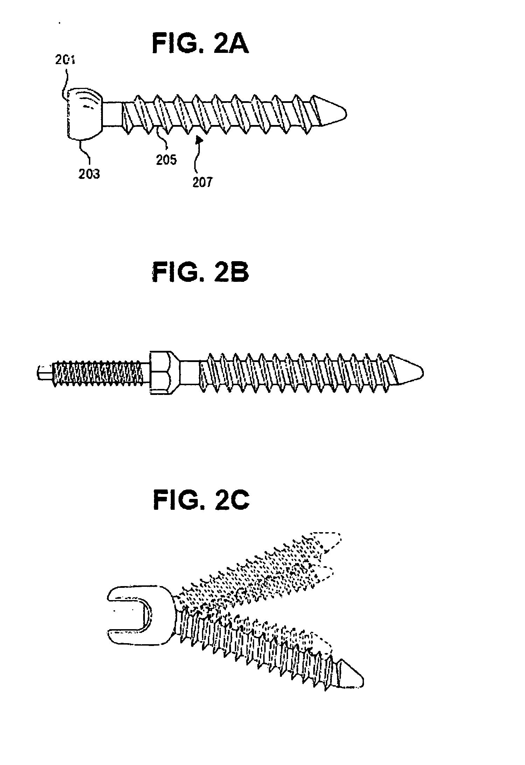

The stabilization devices described herein are self-expanding devices that expand from a compressed profile having a relatively narrow diameter (e.g., a delivery configuration) into an expanded profile (e.g., a deployed configuration). The stabilization devices generally include a shaft region having a plurality of struts that may extend from the shaft body. The distal and proximal regions of a stabilization device may include one or more attachment regions configured to attach to an inserter for inserting (and/or removing) the stabilization device from the body. The stabilization devices described herein may also include one or more bone screw attachment regions to which a bone screw (or screws) may be attached. In general, these devices may be used with any bone screw, including pedicle screws. FIGS. 2A-2E illustrate variations of pedicle screws that may be used with the devices described herein.

In general, inserting bone screws into the pedicles unaided takes a great deal of skill, as the dense bony parts of the pedicle are not large (e.g., pedicle thickness may be 4-6 mm), and a mistake could push a bone fragment into the spinal nerves, causing pain, loss ofmobility and other damage, including damage to major blood vessels. To avoid this risk, the stabilization devices described herein maybe used to position and secure screws, including pedicle screws, into the bone. The devices and method described herein may be used in conjunction with any appropriate visualization technique, including 3-D imaging, to place the stabilization devices into the bone, and then (or concurrently) place and position one or more screws through small incisions in the skin.

In FIG. 2A, the pedicle screw 207 includes a head 203 that may be fixed or adjustable (e.g., rotatable), and may be keyed for use with a drill, screwdriver, or the like 201. A typical pedicle screw may also include a shaft region 205 that is threaded for insertion into the bone. The threads may be any size (e.g., minimum, maximum and pitch). In addition, the bone screw may be any appropriate length or thickness. In some variations, the bone screw has a thickness that is non-uniform along the length of the screw. Similarly, the threading may be non-uniform (e.g., may vary in pitch, maximum, minimum, etc.).

FIG. 2B shows another variation of a pedicle screw that includes a threaded front end, a head, and a threaded back end. This variation of a pedicle screw may be particularly useful in applying additional stabilization materials (e.g., plates, other screws, etc.). For example, in spinal fusion procedures.

Another variation of a one-piece pedicle screw has dual threads. For example, the screw may include a proximal region which has electrical threads (for attachment to the spine stabilization device) and the distal region has wood threads (e.g., in the outer portion that is configured to engage the pedicle). This screw can also be used with a cannula (e.g., canulated) and can have various lengths, pitch, and depths for each type of thread.

In some variations, the distal region of the screw (either a single-component screw of a multi-component screw) is conical, which may allow for ease of re-accessing a spine stabilization device or tissue region.

FIG. 2C is one variation of a polyaxial pedicle screw. This variation of a pedicle screw is threaded and the head is mobile (e.g., it swivels, helping to defray vertebral stress). Like other screws, polyaxial screws come in many sizes. For example, polyaxial pedicle screw length may vary from 30 mm to 60 mm (up to 2-1/2 inches). The diameter may range from 5.0 mm to 8.5 mm (up to 1/4 inch).

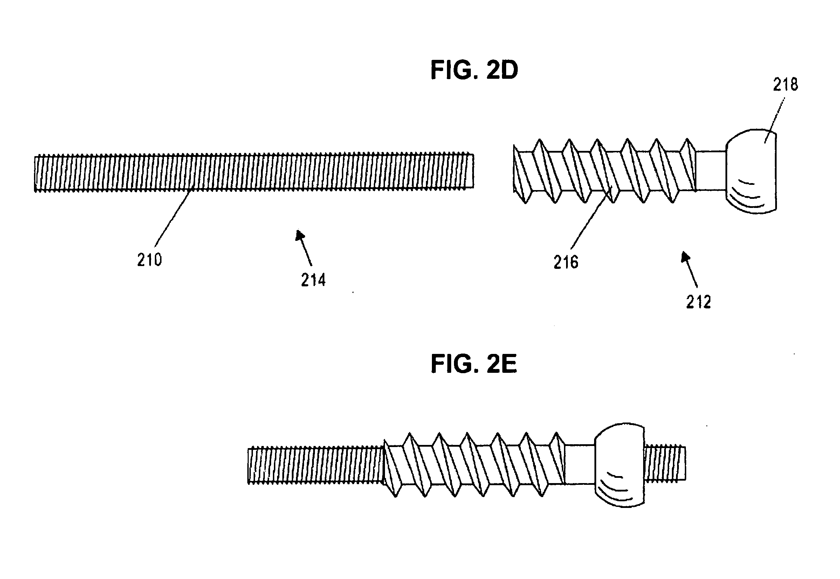

FIG. 2D shows both parts of a two-part bone screw that may be used. This variation of a pedicle screw includes a first bone screw assembly 214 that is configured to mate with the second bone screw assembly 212. In general, a two-part bone screw includes a first assembly that is configured to mate with the stabilization devices described herein, and a second bone screw assembly that is configured to secure to bone, and to mate with the first bone screw assembly. For example, the first bone screw assembly may be threaded 210 along the outer surface. The threads of the outer surface may engage a portion of the stabilization device (described in more detail below). The second bone screw assembly (or outer bone screw assembly) includes an inner region (e.g., threaded region) that engages the outer surface of the first bone screw assembly (not visible in FIG. 2D). In some variations this inner region extends at least partly through the length of the outer bone screw assembly. In other variations, the inner region extends completely through the outer bone screw assembly. The outer surface of the outer bone screw assembly is typically configured to engage bone. For example, the outer surface may be threaded 216, as shown in FIGS. 2D and 2E. In other variations, the outer surface includes other bone-engaging regions, such as protrusions, spurs, etc.

The proximal ends of either or both of the first and second bone screw assemblies of a two-part bone screw may be configured to engage a tool. For example, in FIG. 2D, the proximal end of the second bone screw assembly includes a head region 218. The head region may also be configured to engage an additional component, or it may be mobile, similar to FIG. 2C.

Different-sized first and second bone screw assemblies may be used to form the two-part bone screw. For example, a first bone screw assembly may be configured to mate with second bone screw assemblies having differ outer dimensions, threading pitches, threading heights, lengths, etc. This may allow customization of the two-part bone screw (and any system including them) to better fit a patient.

FIG. 2E illustrates the two-part bone screw of FIG. 2D in which the first and second assemblies are engaged with each other. In this example, the second bone screw assembly has been screwed onto the first bone screw assembly so that the proximal end of the first bone screw assembly extends past the proximal end of the second bone screw assembly.

The distal ends of either (or both) the first and second bone screw assemblies may be configured in any appropriate manner. For example, the distal end of the first bone screw assembly may be configured as a point or taper, as rounded, or as flat. The distal end of the second bone screw assembly is typically configured as opened to mate with the first bone-screw assembly, but this region may also be configured as tapered, smooth, or the like. For example the bone-engaging outer surface (e.g., threads 316) may begin proximal to the distal end, and may increase in size (e.g., depth) along the proximal end of the second bone screw assembly.

The pitch and size of the outer threading of the second bone screw assembly 216 in FIGS. 2D and 2E is generally greater than the pitch and size of the outer threading of the first bone screw assembly. In some variations, the pitch and/or size of these threading is the same, or the pitch and/or size of the outer threading on the first (inner) bone screw assembly is greater than the pitch and/or size of the outer threading on the second (outer) bone screw assembly. It may be particularly advantageous to have a larger pitch and/or larger size threading on the outer bone screw assembly, since it is typically configured to engage bone. The first and second bone screw assemblies may be formed of any appropriate material, including those used for the screws in FIGS. 2A-2C. Appropriately durable biocompatible materials (such as stainless steel) may be particularly appropriate. Coatings (e.g., low-friction coatings, drug coatings, etc.) may also be used. The materials forming the first and second bone screw assemblies may be the same, or they may be different.

In some variations for inserting a multi-part screw and a bone stabilization device, the outer component of the screw may be positioned before the inner component, and then the inner (smaller-diameter) component may be positioned and secured. For example, the multi-part screw may be attached by placing the outer screw first (after placement of the spine stabilizing device) over a pin wire (e.g., guidewire) or over the delivery device used to for the spine stabilization device, and then placing and securing the inter-screw.

The pedicle screws described herein may be any appropriate dimensions and may be made of any appropriate material. For example, the screws may be Titanium, which is highly resistant to corrosion and fatigue, and is MRI compatible.

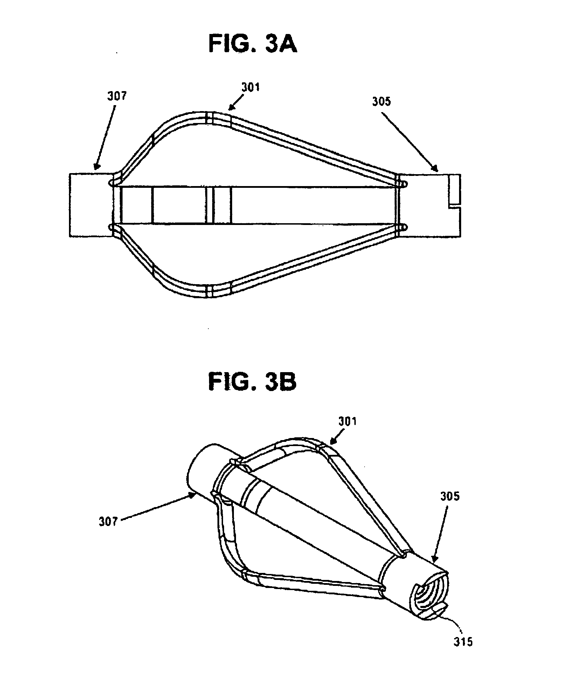

FIGS. 3A through 4B show exemplary stabilization devices that may be used with bone screws as described herein. Side profile views of one variation of a stabilization device are shown in FIGS. 3A and 3B. FIGS. 3A and 3B show a 10 mm asymmetric stabilization device in an expanded configuration. The device has four struts 301 formed by cutting four slots down the length of the shaft. In this example, the elongate expandable shaft has a hollow central lumen, and a proximal end 305 and a distal end 307. By convention, the proximal end is the end closest to the person inserting the device into a subject, and the distal end is the end furthest away from the person inserting the device.

The struts 301 of the elongate shaft is the section of the shaft that projects from the axial (center) of the shaft. Three struts are visible in each of figures 3A and 3B. In general, each strut has a leading exterior surface that forms a cutting surface adapted to cut through cancellous bone as the strut is expanded away from the body of the elongate shaft. This cutting surface may be shaped to help cut through the cancellous bone (e.g., it may have a tapered region, or be sharp, rounded, etc.)). In some variations, the cutting surface is substantially flat.

The stabilization device is typically biased so that it is relaxed in the expanded or deployed configuration, as shown in FIGS. 3A and 3B. In general, force may be applied to the stabilization device so that it assumes the narrower delivery profile. Thus, the struts may elastically bend or flex from the extended configuration to the unextended configuration.

The struts in these examples are continuous curvature of bending struts. Continuous curvature of bending struts are struts that do not bend from the extended to an unextended configuration (closer to the central axis of the device shaft) at a localized point along the length of the shaft. Instead, the continuous curvature of bending struts are configured so that they translate between a delivery and a deployed configuration by bending over the length of the strut rather than by bending at a discrete portion (e.g., at a notch, hinge, channel, or the like). Bending typically occurs continuously over the length of the strut (e.g., continuously over the entire length of the strut, continuously over the majority of the length of the strut (e.g., between 100-90%, 100-80%, 100-70%, etc.), continuously over approximately half the length of the strut (e.g., between about 60-40%, approximately 50%, etc.). In some variations of the self-expanding devices described herein, the struts do not have a continuous curvature of bending, but may be bent or hinged, or may include one or more notches along the length of the strut to facilitate bending.

The "curvature of bending" referred to by the continuous curvature of bending strut is the curvature of the change in configuration between the delivery and the deployed configuration. The actual curvature along the length of a continuous curvature of bending strut may vary (and may even have "sharp" changes in curvature). However, the change in the curvature of the strut between the delivery and the deployed configuration is continuous over a length of the strut, as described above, rather than transitioning at a hinge point. Struts that transition between delivery and deployed configurations in such a continuous manner may be stronger than hinged or notched struts, which may present a pivot point or localized region where more prone to structural failure.

A continuous curvature of bending strut typically does not include one or more notches or hinges along the length of the strut. Two variations of continuous curvature of bending struts are notchless struts and/or hingeless struts. In FIG. 3A, the strut 301 bends in a curve that is closer to the distal end of the device than the proximal end (making this an asymmetric device). In this example, the maximum distance between the struts along the length of device is approximately 10 mm in the relaxed (expanded) state. Thus, this may be referred to as a 10 mm asymmetric device.

The device shown in FIGS. 3A an 3B also include one or more bone screw attachment region(s). For example, in FIG. 3A the bone screw attachment region is located at the proximal 305 end of the shaft. As described in more detail below, this proximal end may also be adapted to releasably engage an inserter for inserting the stabilization device into the bone.

In general a bone screw attachment site is configured to secure a bone screw to the device and therefore into the bone. For example, a bone screw attachment region or site may include an opening or passage into which the bone screw may be inserted. In some variations the bone screw attachment passage is threaded in a manner that is complementary to the bone screw that may be inserted into the device. Thus, in some variations, the bone screw attachment site is configured to mate with a particular size or shape of bone screw (e.g., the size and pitch of threads). In some variations, the bone screw attachment region comprises a passageway that is not threaded, but is configured to be compressed by the bone screw as it is inserted. For example, the bone screw attachment region may include a crushable material (e.g., a porous, frangible, or compressible material) that the bone screw may crush when inserted therethrough.

In some variations, the bone screw attachment region is an opening through which the bone screw may pass, so that the self-expanding stabilization device guides the bone screw insertion. In some variations an adapter or sleeve may be inserted in (or may be present within) the bone screw attachment region of the device. The bone screw may be inserted into the sleeve or adapter. This may allow one size of bone screw attachment region to be used with a variety of differently sized bone screws.

In some variations, the bone screw attachment region is a post or projection connected to or integral with the stabilization device onto which the bone screw (or a portion or component of a bone screw) mates. For example, a bone screw attachment region may be a post projecting from an outer (or inner) surface of the stabilization device over which a bone screw coaxially slides.

The proximal end (the end facing to the right in FIGS. 3A and 3B), shows one variation of an attachment region to which the device may be attached to one portion of an introducer. As mentioned above, this same end of the device may include a bone screw attachment region. The devices shown in FIG. 3A-4B include a bone screw attachment region that passes through this proximal end and into the lumen of the elongate shaft.

In FIG. 3B, the proximal end 305 also includes a cut-out region, forming a seating area into which a complementary attachment region of an inserter may mate. In some variations, this notch (or cut-out region) is not present. In this example, the bone screw attachment region is a threaded region 315 that is visible within the lumen of the shaft. These threads may be used to secure to a bone screw, and/or to releasably secure to an inserter. Thus, the distal region 307 of the device may include an attachment region for attaching the device to an inserter. Alternative or additional bone screw attachment regions may also be included. For example, the distal end of the device may also include a bone screw attachment region.

In addition to the bone screw attachment region(s), the devices described herein may also include an attachment region for relaseably attaching to an inserter. To distinguish the inserter attachment regions from the bone screw attachment regions, bone screw attachment regions may be referred to as "bone screw attachment regions" and inserter attachment regions may be referred to as "inserter attachment regions" or simply as "attachment regions", although in some variations it should be clear that the same regions of the device may be both bone screw attachment regions and inserter attachment regions.

An inserter attachment region may be configured in any appropriate way. For example, the attachment region may be a cut-out region (or notched region), including an L-shaped cut out, an S-shaped cut out, a J-shaped cut out, or the like, into which a pin, bar, or other structure on the inserter may mate. In some variations, the attachment region is a threaded region which may mate with a pin, thread, screw or the like on the inserter. As mentioned, these same regions may also be bone screw attachment regions. In some variations, the inserter attachment region is a hook or latch. The attachment region may be a hole or pit, with which a pin, knob, or other structure on the inserter mates. In some variations, the attachment region includes a magnetic or electromagnetic attachment (or a magnetically permeable material), which may mate with a complementary magnetic or electromagnet region on the inserter. In each of these variations the attachment region on the device mates with an attachment region on the inserter so that the device may be removably attached to the inserter.

The stabilization devices described herein generally have two or more releasable inserter attachment regions for attaching to an inserter. For example, a stabilization device may include at least one inserter attachment region at the proximal end of the device and another inserter attachment region at the distal end of the device. This allows the inserter to apply force across the device (e.g., to pull the device from the expanded deployed configuration into the narrower delivery configuration), as well as to hold the device at the distal end of the inserter. However, the stabilization devices may also have a single attachment region (e.g., at the proximal end of the device). In this variation, the more distal end of the device may include a seating region against which a portion of the inserter can press to apply force to change the configuration of the device. In some variations of the self-expanding stabilization devices, the force to alter the configuration of the device from the delivery to the deployed configuration comes from the material of the device itself (e.g., from a shape-memory material), and thus only a single attachment region (or one or more attachment region at a single end of the device) is necessary.

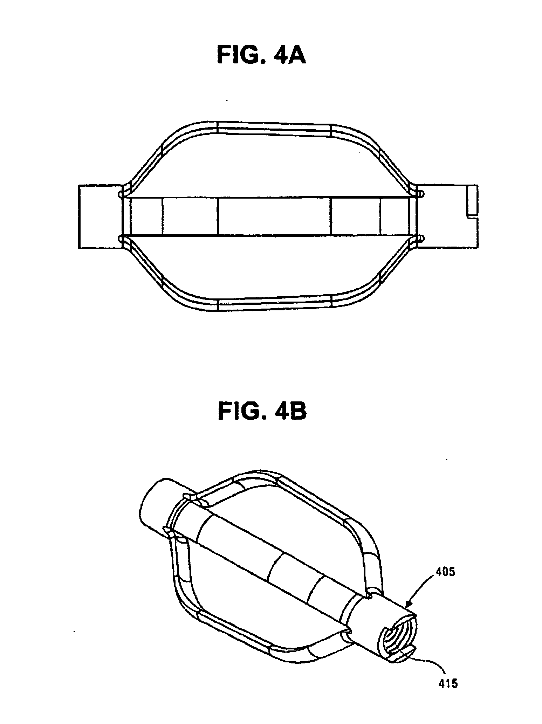

FIGS. 4A and 4B show side and side perspective views of exemplary symmetric 10 mm devices. In FIG. 4B the bone screw attachment region (shown in FIG. 4B as a threaded region 415) is located within the proximal 405 end of the device.

The struts described herein (including the continuous curvature of bending struts) may be any appropriate dimension (e.g., thickness, length, width), and may have a uniform cross-sectional thickness along their length, or they may have a variable cross-sectional thickness along their length. For example, the region of the strut that is furthest from the tubular body of the device when deployed (e.g., the curved region 301 in FIGS. 3A and 3B) may be wider than other regions of the strut, providing an enhanced contacting surface that abuts the non-cancellous bone after deployment.

The dimensions of the struts may also be adjusted to calibrate or enhance the strength of the device, and/or the force that the device exerts to self-expand. For example, thicker struts (e.g., thicker cross-sectional area) may exert more force when self-expanding than thinner struts. This force may also be related to the material properties of the struts.

The struts may be made of any appropriate material. In some variations, the struts and other body regions are made of substantially the same material. Different portions of the stabilization device (including the struts) may be made of different materials. In some variations, the struts may be made of different materials (e.g., they may be formed of layers, and/or of adjacent regions of different materials, have different material properties). The struts may be formed of a biocompatible material or materials. It may be beneficial to form struts of a material having a sufficient spring constant so that the device may be elastically deformed from the deployed configuration into the delivery configuration, allowing the device to self-expand back to approximately the same deployed configuration. In some variation, the strut is formed of a shape memory material that may be reversibly and predictably converted between the deployed and delivery configurations. Thus, a list of exemplary materials may include (but is not limited to): biocompatible metals, biocompatible polymers, polymers, and other materials known in the orthopedic arts. Biocompatible metals may include cobalt chromium steel, surgical steel, titanium, titanium alloys (such as the nickel titanium alloy Nitinol™), tantalum, tantalum alloys, aluminum, etc. Any appropriate shape memory material, including shape memory alloys such as Nitinol™ may also be used.

Other regions of the stabilization device may be made of the same material(s) as the struts, or they may be made of a different material. Any appropriate material (preferably a biocompatible material) may be used (including any of those materials previously mentioned), such as metals, plastics, ceramics, or combinations thereof. In variations where the devices have bearing surfaces (i.e. surfaces that contact another surface), the surfaces may be reinforced. For example, the surfaces may include a biocompatible metal. Ceramics may include pyrolytic carbon, and other suitable biocompatible materials known in the art. Portions of the device can also be formed from suitable polymers include polyesters, aromatic esters such as polyalkylene terephthalates, polyamides, polyalkenes, poly(vinyl) fluoride, PTFE, polyarylethyl ketone, and other materials. Various alternative embodiments of the devices and/or components could comprise a flexible polymer section (such as a biocompatible polymer) that is rigidly or semi rigidly fixed.

For example, the bone screw attachment region may be made of any appropriate material. A bone screw attachment region may include a coating or layer. In particular, materials that help secure the bone screw within the stabilization device or ease the insertion of the bone screw into the device may be used. For example, the bone screw attachment region may include a coating of a friction-reducing material, or a friction-enhancing material. As mentioned above, the bone screw attachment region may include a layer of compressible or crushable material. In some variations the bone screw attachment region comprises a channel for the insertion of a bone screw that is formed of a relatively uncompressible material (e.g., a metal such as steel or titanium) surrounding a relatively compressible material (e.g., aluminum, tin, porous materials, rubbers, frangible materials, etc).

Thus the devices (including the struts), may also include one or more coating or other surface treatment (embedding, etc.). Coatings may be protective coatings (e.g., of a biocompatible material such as a metal, plastic, ceramic, or the like), or they may be a bioactive coating (e.g., a drug, hormone, enzyme, or the like), or a combination thereof. For example, the stabilization devices may elute a bioactive substance to promote or inhibit bone growth, vascularization, etc. In one variation, the device includes an elutible reservoir of bone morphogenic protein (BMP).

As previously mentioned, the stabilization devices may be formed about a central elongate hollow body. In some variations, the struts are formed by cutting a plurality of slits long the length (distal to proximal) of the elongate body. This construction may provide one method of fabricating these devices, however the stabilization devices are not limited to this construction. If formed in this fashion, the slits may be cut (e.g., by drilling, laser cutting, etc.) and the struts formed by setting the device into the deployed shape so that this configuration is the default, or relaxed, configuration in the body. For example, the struts may be formed by plastically deforming the material of the struts into the deployed configuration. In general, any of the stabilization devices may be thermally treated (e.g., annealed) so that they retain this deployed configuration when relaxed. Thermal treatment may be particularly helpful when forming a strut from a shape memory material such as a nickel-titanium alloy (e.g., Nitinol™) into the deployed configuration.

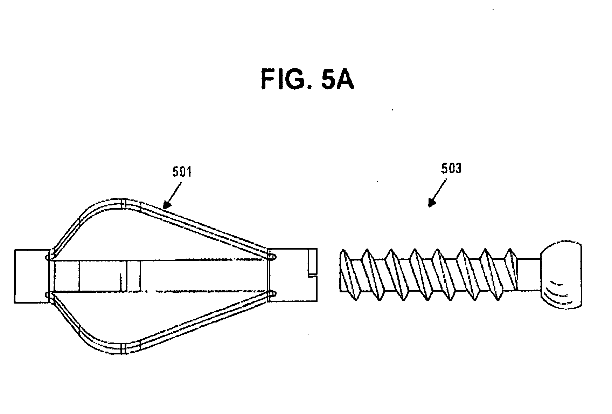

FIG. 5A shows one variation of a stabilization device 501 including a bone screw attachment region. A bone screw 503 is shown attached to the stabilization device 501. In this example, the bone screw attachment region is located at the proximal end of the device. The device 501 includes a hollow body into which the bone screw may be inserted. In use, the bone screw may be inserted into the device either before, during, or after the device has been inserted into the subject's bone.

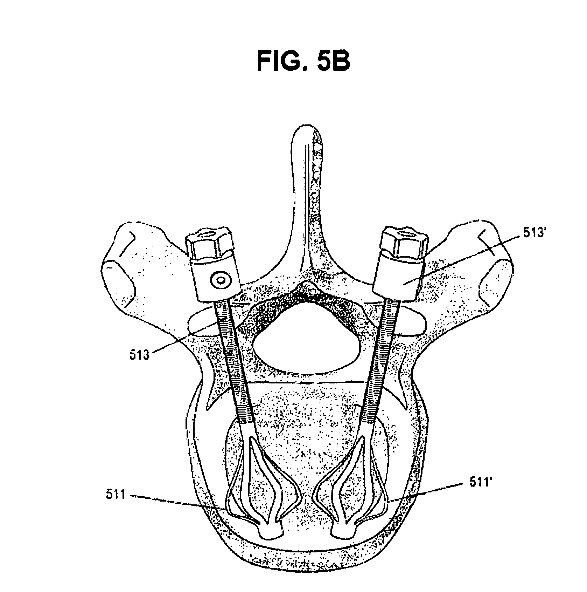

FIG. 5B illustrates two stabilization devices 511, 511' inserted into a spinal segment. A pedicle (bone) screw 513, 513' has been inserted into each stabilization device. In some variations, the distal end of the device may also include a bone screw attachment region, so that a pedicle screw may be stabilized both at the proximal and the distal ends of the device. Thus, a bone screw may be inserted completely through the stabilization device, and may extend from the distal end. In some variations, the central region of the device includes a continuous (or mostly continuous) channel into which the bone screw may pass.

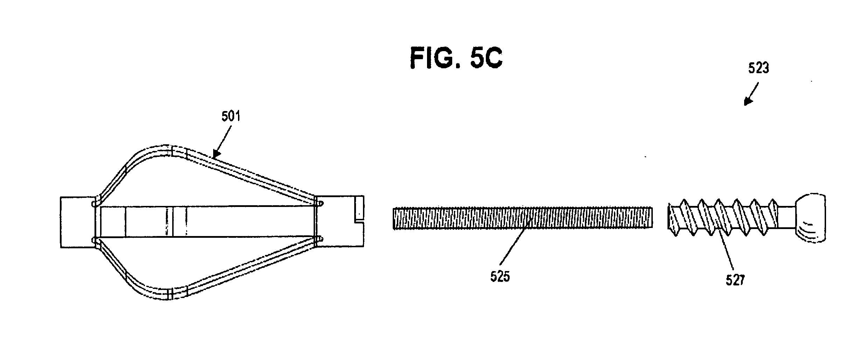

FIG. 5C shows another variation of a system including a stabilization device 501 having a bone screw attachment region and a bone screw 523. The bone screw in this example is a two-part bone screw 523 in which the first bone screw assembly 525 attaches to the stabilization device 501, and the second bone screw assembly 527 attaches to the first bone screw assembly. In some variations, the second bone screw assembly may also attach to the stabilization device 501. In use, the first and second bone screw assemblies may be inserted into the device, and/or may engage with each other either before, during, or after the device has been inserted into the subject's bone.

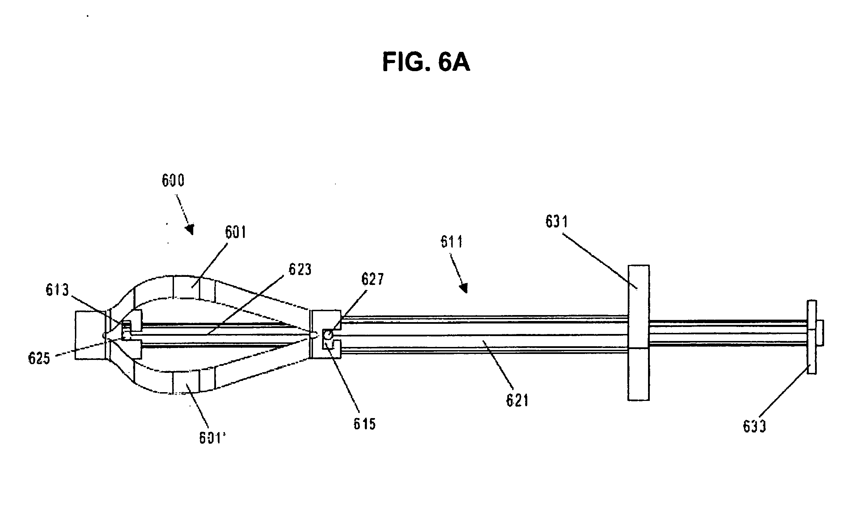

Any of the stabilization device described herein are used with an inserter that may position the self-expanding stabilization device within the subject's bone. FIG. 6A shows one variation of a stabilization device 600 having a plurality of continuous curvature of bending struts 601, 601' removably attached to an inserter 611. This stabilization device also includes at least one bone screw attachment device. In this example, an inserter attachment region 615 at the proximal portion of the stabilization device is configured as an L-shaped notch, as is the attachment region 613 at the distal portion of the device.

In general, an inserter includes an elongate body having a distal end to which the stabilization device may be attached and a proximal end which may include a handle or other manipulator that coordinates converting an attached stabilization device from a delivery and a deployed configuration, and also allows a user to selectively release the stabilization device from the distal end of the inserter.

The inserter 611 shown in FIG. 6A includes a first elongate member 621 that coaxially surrounds a second elongate member 623. In this variation, each elongate member 621, 623 includes a stabilization device attachment region at its distal end, to which the stabilization device is attached, as shown. In this example, the stabilization device attachment region includes a pin that mates with the L-shaped slots forming the releasable attachment regions on the stabilization device. In FIG. 6A the L-shaped releasable attachments on the stabilization device are oriented in opposite diiections (e.g., the foot of each "L" points in opposite directions). Thus, the releasable attachment devices may be locked in position regardless of torque applied to the inserter, preventing the stabilization device from being accidentally disengaged.

The inserter shown in FIG. 6A also includes two grips 631, 633 at the proximal ends of each elongate member 621, 623. These grips can be used to move the elongate members (the first 621 or second 623 elongate member) relative to each other. The first and second elongate members of the inserter may be moved axially (e.g., may be slid along the long axis of the inserter) relative to each other, and/or they may be moved in rotation relative to each other (around the common longitudinal axis). Thus, when a stabilization device is attached to the distal end of the inserter, moving the first elongate member 621 axially with respect to the second elongate member 623 will cause the stabilization device to move between the deployed configuration (in which the struts are expanded) and the delivery configuration (in which the struts are relatively unexpanded). Furthermore, rotation of the first elongate member of the inserter relative to the second elongate member may also be used to disengage one or more releasable attachment regions of the stabilization device 613, 615 from the complementary attachment regions of the inserter 625, 627. The inserter is used to apply additional force to convert the stabilization device between the delivery and the deployed configuration. For example, when allowed to expand in a cancellous bone, the force applied by the struts when self-expanding may not be sufficient to completely cut through the cancellous bone and/or distract the cortical bone as desired. In some variations, the inserter may also permit the application of force to the stabilization device to expand the struts even beyond the deployed configuration.

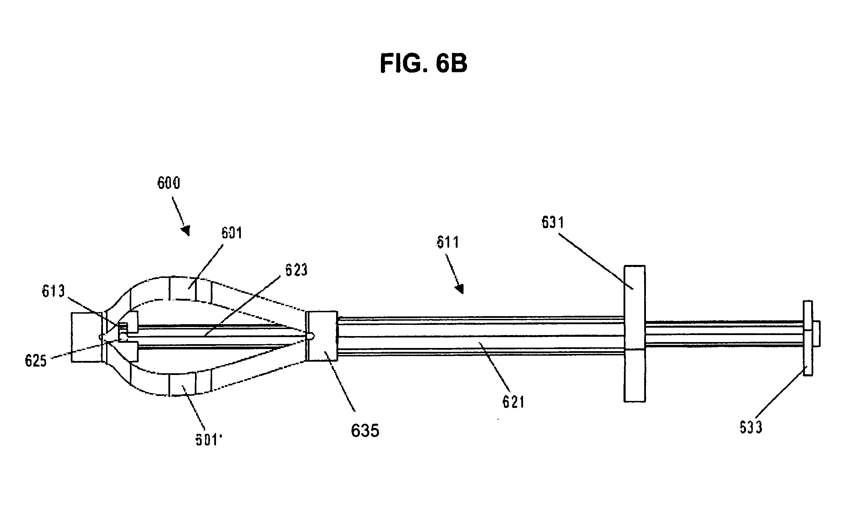

FIG. 6B is another variation of a stabilization device 600 releasably connected to an inserter 611, in which the attachment region 635 between the stabilization device and the inserter is configured as a screw or other engagement region, rather than the notch 615 shown in FIG. 6A.

An inserter may also limit or guide the movement of the first and second elongate members, so as to further control the configuration and activation of the stabilization device. For example, the inserter may include a guide for limiting the motion of the first and second elongate members. A guide may be a track in either (or both) elongate member in which a region of the other elongate member may move. The inserter may also include one or more stops for limiting the motion of the first and second elongate members.

As mentioned above, the attachment regions on the inserter mate with the stabilization device inserter attachments. Thus, the attachment regions of the inserter may be complementary attachments that are configured to mate with the stabilization device inserter attachments. For example, a complimentary attachment on an inserter may be a pin, knob, or protrusion that mates with a slot, hole, indentation, or the like on the stabilization device. In some variations the attachment region is a threaded region. The complementary attachment (the attachment region) of the inserter may be retractable. For example, the inserter may include a button, slider, etc. to retract the complementary attachment so that it disconnects from the stabilization device attachment. A single control may be used to engage/disengage all of the complementary attachments on an inserter, or they may be controlled individually or in groups.

In some variation the inserter includes a lock or locks that hold the stabilization device in a desired configuration. For example, the inserter may be locked so that the stabilization device is held in the delivery configuration (e.g., by applying force between the distal and proximal ends of the stabilization device). In an inserter such as the one shown in FIG. 6A, for example, a lock may secure the first elongate member to the second elongate member so that they may not move axially relative to each other.

In some variations, the inserter includes a space or passage for a bone screw that may be pre-attached to the stabilization device before the device is implanted. The inserter may also include a holder (or holding region) for a bone screw, and the inserter may be used to attach the bone screw to a stabilization device after it has been implanted.

Any of the inserters described herein may include, or may be used with, a handle. A handle may allow a user to control and manipulate an inserter. For example, a handle may conform to a subject's hand, and may include other controls, such as triggers or the like. Thus, a handle may be used to control the relative motion of the first and second elongate members of the inserter, or to release the connection between the stabilization device and the inserter, or any of the other features of the inserter described herein.

An inserter may be packaged or otherwise provided with a stabilization device attached. Thus, the inserter and stabilization device may be packaged sterile, or may be sterilizable. In some variations, a reusable handle is provided that may be used with a pre-packaged inserter stabilization device assembly.

Any of the stabilization device including bone screw attachment sites may also be included with a bone screw or screws. Thus a system or kit including a stabilization device may also include one or more bone screws.

As mentioned above, in the delivery configuration the struts of the stabilization device are typically closer to the long axis of the body of the stabilization device. Thus, the device may be inserted into the body for delivery into a bone region. This may be accomplished with the help of an access cannula (which may also be referred to as an introducer). Any of the devices (stabilization devices) and inserters (including handles) may be included as part of a system or kit for correcting a bone defect or injury. A trocar may also be used with an access cannula to insert the devices. Any appropriate length cannula and trocar may be used, so long as it is correctly scaled for use with the introducer and stabilization device. A trocar and an introducer may be used to cut through tissue until reaching bone, so that the introducer can be positioned appropriately.

A bone drill may be used to access the cancellous bone to insert any of the devices described. A twist drill may be used with the same access cannula previously described. The distal (drill) end of the twist drill may extend from the cannula, and be used to drill into the bone.

Any of the devices shown and described herein may also be used with a bone cement. For example, a bone cement may be applied after inserting the stabilization device into the bone, before or after attaching a bone screw. Bone cement may be used to provide long-term support for the repaired bone region and bone screw. Any appropriate bone cement or filler may be used, including PMMA, bone filler or allograft material. Suitable bone filler material include bone material derived from demineralized allogenic or xenogenic bone, and can contain additional substance, including active substance such as bone morphogenic protein (which induce bone regeneration at a defect site). Thus materials suitable for use as synthetic, non-biologic or biologic material may be used in conjunction with the devices described herein, and may be part of a system includes these devices. For example, polymers, cement (including cements which comprise in their main phase of microcrystalline magnesium ammonium phosphate, biologically degradable cement, calcium phosphate cements, and any material that is suitable for application in tooth cements) may be used as bone replacement, as bone filler, as bone cement or as bone adhesive with these devices or systems. Also included are calcium phosphate cements based on hydroxylapatite (HA) and calcium phosphate cements based on deficient calcium hydroxylapatites (CDHA, calcium deficient hydroxylapatites). See, e.g., U.S. Pat. No. 5,405,390 to O'Leary et al.; U.S. Pat. No. 5,314,476 to Prewett et al.; U.S. Pat No. 5,284,655 to Bogdansky et al.; U.S. Pat. No. 5,510,396 to Prewett et al.; U.S. Pat. No. 4,394,370 to Jeffries; and U.S. Pat. No. 4,472,840 to Jeffries, which describe compositions containing demineralized bone powder. See also U.S. Pat. No. 6,340,477 to Anderson which describes a bone matrix composition.

### Exemplary Method of Repairing a Bone

As mentioned above, any of the devices described herein may be used to repair a bone. A method not claimed, of treating a bone using the devices describe herein typically involves delivering a stabilization device (e.g., a self-expanding stabilization device as described herein including a bone screw attachment region) within a cancellous bone region, and allowing the device to expand within the cancellous bone region so that a cutting surface of the device cuts through the cancellous bone.

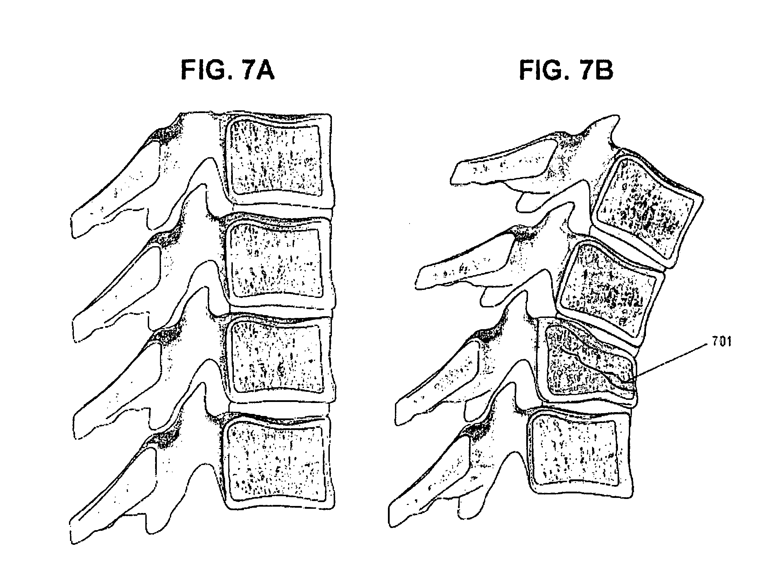

For example, the stabilization devices described herein may be used to repair a compression fracture in spinal bone. This is illustrated schematically in FIGS. 7A-7J. FIG. 7A shows a normal thoracic region of the spine in cross-section along the sagital plane. The spinal vertebras are aligned, distributing pressure across each vertebra. FIG. 7B shows a similar cross-section through the spine in which there is a compression fracture in the 11^{th} thoracic vertebra 701. The 11^{th} vertebra is compressed in the fractured region. It would be beneficial to restore the fractured vertebra to its uninjured position, by expanding (also referred to as distracting) the vertebra so that the shape of the cortical bone is restored. It may also be useful to insert a pedicle (bone) screw so that this spinal region can be fused. This may be achieved by inserting and expanding one of the stabilization devices described herein, and then attaching a pedicle screw. In order to insert the stabilization device, the damaged region of bone must be accessed.

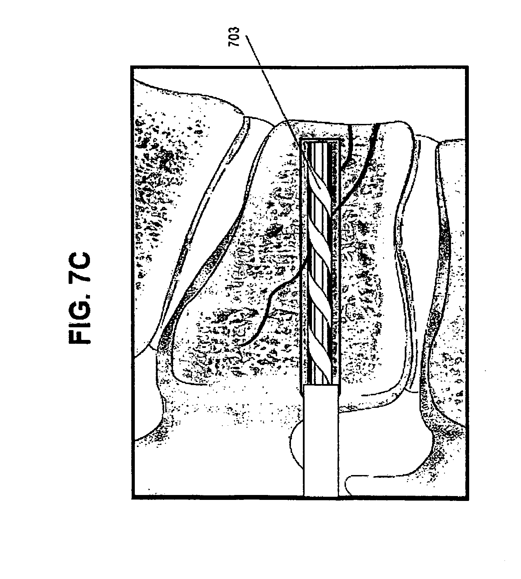

As mentioned above, an introducer (or access cannula) and a trocar may be used to insert the access cannula adjacent to the damaged bone region. Any of the steps described herein may be aided by the use of an appropriate visualization technique. For example, a fluoroscope may be used to help visualize the damaged bone region, and to track the process of inserting the access cannula, trocar, and other tools. Once the access cannula is near the damaged bone region, a bone drill may be used to drill into the bone, as shown in FIG. 7C.

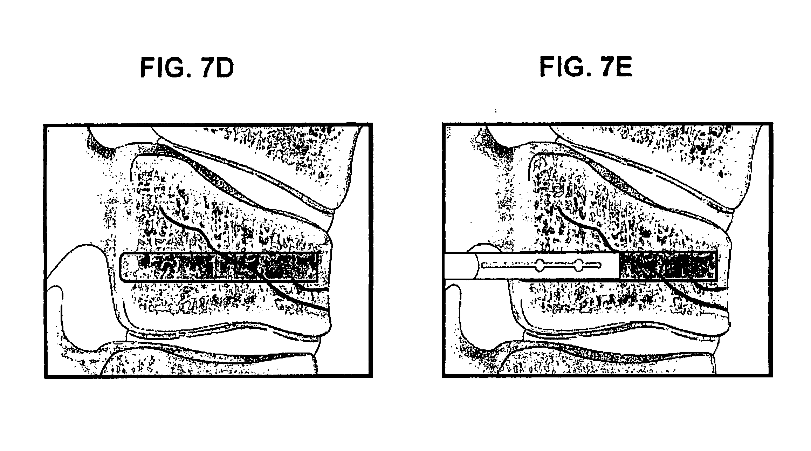

In FIG. 7C the drill 703 enters the bone from the access cannula. The drill enters the cancellous bony region within the vertebra. After drilling into the vertebra to provide access, the drill is removed from the bone and the access cannula is used to provide access to the damaged vertebra, as shown, by leaving the access cannula in place, providing a space into which the stabilization device may be inserted in the bone, as shown in FIG. 7D. In FIG. 7E a stabilization device, attached to an inserter and held in the delivery configuration, is inserted into the damaged vertebra.

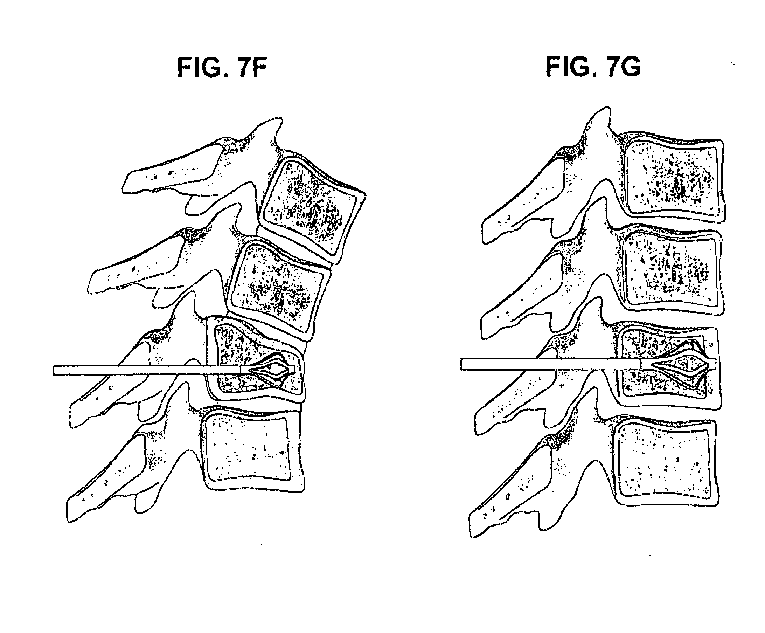

Once in position within the vertebra, the stabilization device is allowed to expand (by self-expansion) within the cancellous bone of the vertebra, as shown in FIG. 7F. In some variations, the device may fully expand, cutting through the cancellous bone and pushing against the cortical bone with a sufficient restoring force to correct the compression, as shown in FIG. 7G. However, in some variations, the force generated by the device during self-expansion is not sufficient to distract the bone, and the inserter handle may be used (e.g., by applying force to the handle, or by directly applying force to the proximal end of the inserter) to expand the stabilization device until the cortical bone is sufficiently distracted.

After the stabilization device has been positioned and is expanded, it may be released from the inserter. In some variations, it may be desirable to move or redeploy the stabilization device, or to replace it with a larger or smaller device. If the device has been separated from the inserter (e.g., by detaching the removable attachments on the stabilization device from the cooperating attachments on the inserter), then it may be reattached to the inserter. Thus, the distal end of the inserter can be coupled to the stabilization device after implantation. The inserter can then be used to collapse the stabilization device back down to the delivery configuration (e.g., by compressing the handle in some variation), and the device can be withdrawn or re-positioned.

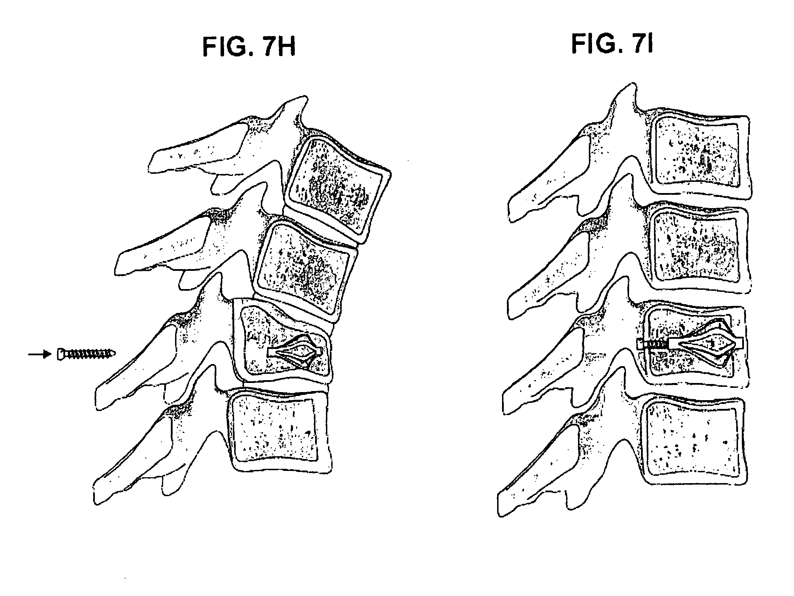

Once the device has been correctly positioned in the bone, a bone screw may be inserted in the same pathway formed to insert the stabilization device, as shown in FIG. 7H (arrow). The bone screw may engage the bone screw attachment region of the stabilization device and be secured by the stabilization device within the bone. As previously mentioned, bone cement may also be used (e.g., inserted into the bone stabilization device prior to adding the bone screw) to further stabilize the bone screw. Once the bone screw has engaged the stabilization device in the bone screw attachment region, it may be secured in position (e.g., by screwing). As shown in FIG. 7I, the bone screw is then secured in the bone by virtue of the association with the stabilization device.

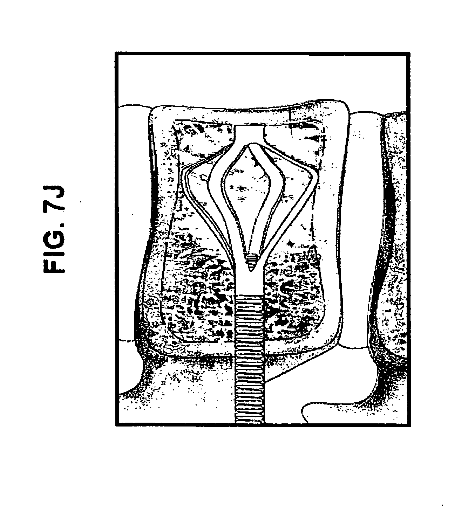

In some variations, a cement (e.g., a bone cement) may also be added to the stabilization device, as shown in FIG. 7J. As briefly mentioned above, the cement may be added either before, during, or after the attachment of a bone screw.

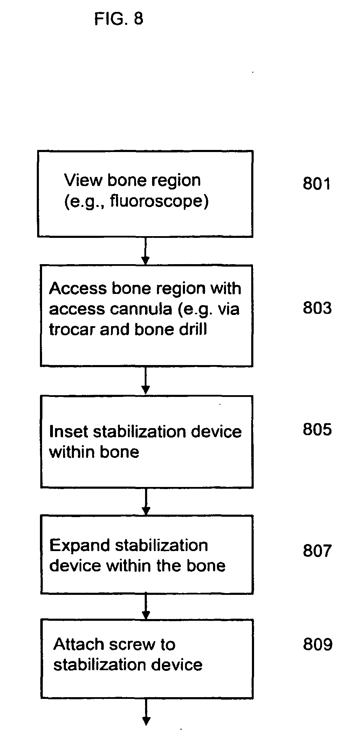

FIG. 8 shows a flowchart summarizing a method, not claimed, for repairing a bone, as described herein. For example, the bone region into which the stabilization device and bone screw are to be inserted may first be visualized (e.g., using a fluoroscope or other appropriate visualization technique) 801. The bone region can then be accessed using an access cannual (e.g., a trocar and/or drill) 803 to form a cavity into which the stabilization device can be inserted. The location (e.g., position, depth, etc.) of the stabilization device can be determined with respect to the eventual addition of the bone screw. Thus, the angle into which the stabilization device is inserted my partially determine the angle that the proximal end of the bone screw presents. Once the bone has been prepared, the stabilization device can be inserted 805 into the bone, and allowed to expand 807. As mentioned, additional force may be applied to help position and further expand the device. Once the device is expanded, additional support material (e.g., fluent bone cement, etc.) may be added if desired. A bone screw may then be attached by mating the bone screw with the bone screw attachment region of the self-expanding stabilization device 809. The final depth and position of the bone screw (as well as any additional structures such as plates, screws, etc.) maybe adjusted by altering the level to which the bone screw is inserted into the stabilization device, etc.

The methods, not claimed, described herein outline only one example of the use of the devices described herein, and additional variations may be included. While embodiments of the present invention have been shown and described herein, such embodiments are provided by way of example only. Thus, alternatives to the embodiments of the invention described herein may be employed in practicing the invention.

## Claims

1. A system for stabilizing a vertebral body, the system comprising:
a self-expanding stabilization device (600) having an elongate shaft, a bone screw attachment region, and a plurality of struts (601, 601') extending therefrom, the stabilization device configured to expand from a compressed delivery configuration to an expanded deployed configuration; and
an inserter (611) having a first stabilization device attachment region (627), adapted to releasably secure to a proximal region of the stabilization device, and a second stabilization device attachment region (625) adapted to secure to a distal region of the stabilization device,
wherein the inserter is adapted to apply a force across the stabilization device to hold it in the compressed delivery configuration and to release the force to allow the stabilization device to expand.

2. The system of claim 1, further comprising a bone screw (503, 513, 523).

3. The system of claim 2, wherein the bone screw comprises a multi-part bone screw (523) comprising:
a first elongate bone screw assembly (525) that is threaded along the outer surface; and
a second elongate bone screw assembly. (527) having a threaded outer surface and an inner channel configured to mate with the outer threads of the first bone screw assembly.

4. The system of any one of claims 1 to 3, further comprising an introducer.

5. The system of any one of claims 1 to 4, further comprising a handle.

6. The system of any one of the preceding claims, wherein the bone screw attachment region comprises a threaded region.

7. The system of claim 6, wherein the threaded region is located at one end of the elongate shaft.

8. The system of any one of the preceding claims, further comprising a second bone screw attachment region.

9. The system of any one of claims 1 to 5, wherein the bone screw attachment region is a threaded opening at the proximal end of the stabilization device.

10. The system of any one of claims 1 to 5, wherein the bone screw attachment region comprises a post onto which the bone screw attaches.

11. The system of any one of the preceding claim, wherein the struts (601, 601') are formed of a shape memory alloy.

12. The system of any one of the preceding claims, further comprising a first releasable attachment (613) having an L-shaped notch.

13. The system of any one of claims 1 to 11, further comprising a first releasable attachment having a threaded region.

14. The system of claim 12 or 13, further comprising a second releasable attachment (615) having an L-shaped notch.

15. The device of any one of the preceding claims, wherein the maximum, distance between the struts (601, 601') at a point along the length of the shaft in the expanded deployed configuration is between about 0.5 and about 30 mm.

## Patentansprüche

1. System zum Stabilisieren eines Wirbelkörpers, wobei das System umfasst:
eine selbstexpandierende Stabilisierungseinrichtung (600) mit einem länglichen Schaft, einem Knochenschraubenbefestigungsbereich und einer Vielzahl von Streben (601, 601'), die sich von diesem weg erstrecken, wobei die Stabilisierungseinrichtung ausgebildet ist, von einer komprimierten zuführungskonfiguration zu einer expandierten eingesetzten Konfiguration zu expandieren; und
eine Einsetzvorrichtung (611) mit einem ersten Befestigungsbereich (627) für die Stabilisierungseinrichtung, der an einem proximalen Bereich der Stabilisierungseinrichtung lösbar befestigbar ist, und mit einem zweiten Befestigungsbereich (625) für die Stabilisierungseinrichtung, der an einem distalen Bereich der Stabilisierungseinrichtung befestigbar ist,
wobei die Einsetzvorrichtung geeignet ist, durch die Stabilisierungseinrichtung hindurch eine Kraft aufzubringen, um sie in der komprimierten Zuführungskonfiguration zu halten, und die Kraft wegzunehmen, und das Expandieren der Stabilisierungseinrichtung zu ermöglichen.

2. System nach Anspruch 1, welches ferner eine Knochenschraube (503, 513, 523) umfasst.

3. System nach Anspruch 2, wobei die Knochenschraube eine mehrteilige Knochenschraube (523) umfasst, die aufweist:
eine erste längliche Knochenschraubenanordnung (525), die längs ihrer äußeren Oberfläche mit einem Gewinde versehen ist, und
eine zweite längliche Knochenschraubenanordnung (527), die eine äußere Gewindeoberfläche und einen inneren Kanal aufweist, der derart ausgebildet ist, dass er mit dem äußeren Gewinde der ersten Knochenschraubenanordnung zusammenpasst.

4. System nach einem der Ansprüche 1 bis 3, das ferner eine Einführungsvorrichtung umfasst.

5. System nach einem der Ansprüche 1 bis 4, das ferner eine Handhabe umfasst.

6. System nach einem der vorhergehenden Ansprüche, wobei der Knochenschraubenbefestigungsbereich einen Gewindebereich umfasst.

7. System nach Anspruch 6, wobei der Gewindebereich an einem Ende des länglichen Schafts angeordnet ist.

8. System nach einem der vorhergehenden Ansprüche, das ferner einen zweiten Knochenschraubenbefestigungsbereich umfasst.

9. System nach einem der Ansprüche 1 bis 5, wobei der Knochenschraubenbefestigungsbereich eine mit einem Gewinde versehene Öffnung am proximalen Ende der Stabilisierungseinrichtung ist.

10. System nach einem der Ansprüche 1 bis 5, wobei der Knochenschraubenbefestigungsbereich eine Stange umfasst, auf der die Knochenschraube befestigt ist.

11. System nach einem der vorhergehenden Ansprüche, wobei die Streben (601, 601') aus einer Formgedächtnislegierung gebildet sind.

12. System nach einem der vorhergehenden Ansprüche, das ferner eine erste lösbare Befestigung (613) mit einer L-förmigen Einkerbung umfasst.

13. System nach einem der Ansprüche 1 bis 11, das ferner eine erste lösbare Befestigung mit einem Gewindebereich umfasst.

14. System nach Anspruch 12 oder 13, das ferner eine zweite lösbare Befestigung (615) mit einer L-förmigen Einkerbung umfasst.

15. Vorrichtung nach einem der vorhergehenden Ansprüche, wobei der maximale Abstand zwischen den Streben (601, 601') an einem Punkt entlang der Länge des Schafts in der expandierten eingesetzten Konfiguration zwischen etwa 0,5 und etwa 30 mm beträgt.

## Revendications

1. Système de stabilisation d'un corps vertébral, le système comprenant :
un dispositif de stabilisation auto-extensible (600) ayant un arbre allongé, une zone de fixation de vis à os et une pluralité d'entretoises (601, 601') s'étendant à partir de celle-ci, le dispositif de stabilisation étant configuré de façon à s'étendre d'une configuration de distribution comprimée à une configuration déployée étendue ; et
un système d'insertion (611) ayant une première zone de fixation du dispositif de stabilisation (627), adaptée pour être fixée de manière amovible à une zone proximale du dispositif de stabilisation, et une seconde zone de fixation du dispositif de stabilisation (625) adaptée pour être fixée à une zone distale du dispositif de stabilisation,
dans lequel le dispositif d'insertion est adapté pour appliquer une force à travers le dispositif de stabilisation afin de le maintenir dans la configuration de distribution comprimée et pour relâcher la force afin de permettre l'extension du dispositif de stabilisation.

2. Système selon la revendication 1, comprenant en outre une vis à os (503, 513, 523).

3. Système selon la revendication 2, dans lequel la vis à os comprend une vis à os à multiples parties (523) comprenant :
un premier ensemble de vis à os allongé (525) qui est fileté le long de la surface extérieure ; et
un second ensemble de vis à os allongé (527) ayant une surface extérieure filetée et un canal interne configuré pour s'adapter aux filets extérieurs du premier ensemble de vis à os.

4. Système selon l'une quelconque des revendications 1 à 3, comprenant en outre un introducteur.

5. Système selon l'une quelconque des revendications 1 à 4, comprenant en outre une poignée.

6. Système selon l'une quelconque des revendications précédentes, dans lequel la zone de fixation de vis à os comprend une zone filetée.

7. Système selon la revendication 6, dans lequel la zone filetée est située à une extrémité de l'arbre allongé.

8. Système selon l'une quelconque des revendications précédentes, comprenant en outre une seconde zone de fixation de vis à os.

9. Système selon l'une quelconque des revendications 1 à 5, dans lequel la zone de fixation de la vis à os est une ouverture filetée à l'extrémité proximale du dispositif de stabilisation.

10. Système selon l'une quelconque des revendications 1 à 5, dans lequel la zone de fixation de la vis à os comprend un montant sur lequel se fixe la vis à os.

11. Système selon l'une quelconque des revendications précédentes, dans lequel les entretoises (601, 601') sont formées d'un alliage à mémoire de forme.

12. Système selon l'une quelconque des revendications précédentes, comprenant en outre une première fixation amovible (613) ayant une encoche en forme de L.

13. Système selon l'une quelconque des revendications 1 à 11, comprenant en outre une première fixation amovible ayant une zone filetée.

14. Système selon la revendication 12 ou 13, comprenant en outre une seconde fixation amovible (615) ayant une encoche en forme de L.

15. Dispositif selon l'une quelconque des revendications précédentes, dans lequel la distance maximale entre les entretoises (601, 601'), en un point situé le long de la longueur de l'arbre, dans la configuration déployée étendue, est comprise entre environ 0,5 et environ 30 mm.
